# EUROPÄISCHE PATENTSCHRIFT

(11) **EP 1 194 531 B1**
(45) Veröffentlichungstag und Bekanntmachung des Hinweises auf die Patenterteilung: **12.05.2004**
(21) Anmeldenummer: 00951406.8
(22) Anmeldetag: 19.07.2000
(51) Int. Cl.: C12N 9/72, A61K 38/49, A61P 35/00

(54) **ZYKLISCHE PEPTIDOMIMETISCHE UROKINASEREZEPTORANTAGONISTEN**
CYCLIC PEPTIDOMIMETIC UROKINASE RECEPTOR ANTAGONISTS
ANTAGONISTES DU RECEPTEUR D'UROKINASE CYCLIQUES PEPTIDOMIMETIQUES

(30) Priorität: 19.07.1999 DE 19933701
(43) Veröffentlichungstag der Anmeldung: 10.04.2002
(73) Patentinhaber: Wilex AG, 81675 München (DE)
(72) Erfinder: WILHELM, Olaf, D-81545 München (DE); KESSLER, Horst, D-85748 Garching (DE); BÜRGLE, Markus, D-81369 München (DE); POTTHOFF, Nils, D-85221 Dachau (DE); SCHMIEDEBERG, Niko, D-81679 München (DE)
(74) Vertreter: Weiss, Wolfgang, Dipl.-Chem. Dr.
(86) Internationale Anmeldenummer: PCT/EP2000/006905
(87) Internationale Veröffentlichungsnummer: WO 2001/005811

(56) Entgegenhaltungen:
- WO-A-97/12905
- WO-A-98/21230
- WO-A-98/46632
- MAGDOLEN V ET AL: "SYSTEMATIC MUTATIONAL ANALYSIS OF THE RECEPTOR-BINDING REGION OF THE HUMAN UROKINASE-TYPE PLASMINOGEN ACTIVATOR" EUROPEAN JOURNAL OF BIOCHEMISTRY,DE,BERLIN, Bd. 237, 1996, Seiten 743-751, XP000652373 ISSN: 0014-2956

## Beschreibung

Die vorliegende Erfindung betrifft zyklische Peptide als Inhibitoren der Bindung von Urokinase an den Urokinaserezeptor, die als pharmazeutische Wirkstoffe für Krankheiten geeignet sind, die durch Urokinase und ihren Rezeptor vermittelt werden. Die erfindungsgemäßen Substanzen sind von der uPA-Sequenz abgeleitete Peptide und entfalten als Liganden des Urokinaserezeptors (uPAR) eine antagonistische Wirkung, und werden im folgenden als uPAR-Antagonisten bezeichnet.

Die Serinprotease uPA (Urokinase-Typ-Plasminogenaktivator) ist für verschiedene physiologische und pathologische Prozesse verantwortlich, wie etwa den proteolytischen Abbau von extrazellulärem Matrixmaterial, das für die Invasibilität und Wanderung von Zellen sowie für die Geweberemodellierung notwendig ist. uPA bindet mit hoher Affinität (K_{D} = 10⁻¹⁰-10⁻⁹M) an den membranständigen uPA Rezeptor (uPAR) auf der Zelloberfläche.

Die Bindung von uPA an seinen Rezeptor ist an vielen invasiven biologischen Prozessen wie etwa der Metastasierung bösartiger Tumoren, der Trophoplastenimplantation, Entzündungen und Angiogenese beteiligt. Daher sind Antagonisten von uPAR in der Lage, die Invasivität, Metastasierung und Angiogenese von Tumoren zu hemmen. uPAR-Antagonisten können als Mittel zur Therapie invasiver und metastasierender Krebserkrankungen eingesetzt werden, bei denen uPA und uPAR an den invasiven Foci von Tumoren auftreten (Dano et al.: The receptor for urokinase plasminogen activator: Stromal cell involvement in extracellular proteolysis during cancer invasion, in: Proteolysis and Protein Turnover, Barrett, A. J. und Bond, J., HRSG, Portland Press, London, 1994, 239), z. B. bei Krebserkrankungen von Brust, Lunge, Darm und Ovarien. Darüber hinaus können uPAR-Antagonisten auch für andere Zwecke eingesetzt werden, bei denen eine Hemmung der proteolytischen Aktivierung von Plasminogen erforderlich ist, beispielsweise zur Bekämpfung von Krankheiten wie Arthritis, Entzündungen, Osteoporose, Retinopathien und zur Kontrazeption.

Der uPA Rezeptor ist in WO 90/ 12091 sowie in den Veröffentlichungen Ploug et al., J. Biol. Chem. 268 (1993), 17539 und Ronne et al., J. Immunol. Methods 167 (1994), 91 beschrieben.

uPA wird als einzelkettiges Molekül (pro-uPA) synthetisiert und enzymatisch in einen aktiven zweikettigen uPA überführt. Das uPA Molekül besteht aus drei strukturell unabhängigen Domänen, der N-terminal lokalisierten wachstumsfaktorartigen Domäne (GFD, uPA 1 - 46), einer Kringelstrukturdomäne (uPA 45 - 135) und der Serinproteasedomäne (uPA 159 - 411). GFD und die Kringeldomäne bilden zusammen das sogenannte aminoterminale Fragment von uPA (ATF, uPA 1 - 135), das durch weitere proteolytische Spaltung von zweikettigem uPA erzeugt wird. ATF bindet an den uPA Rezeptor mit einer ähnlichen Affinität wie uPA.

Die rezeptorbindende Region von uPA erstreckt sich über den Bereich der Aminosäuren 12 bis 32, da ein Peptid, welches die Aminosäurereste 12 bis 32 von uPA enthält (wobei Cystein an Position 19 durch Alanin ersetzt ist) mit ATF um die Bindung an den uPA Rezeptor kompetiert (Appella et al., J. Biol. Chem. 262 (1987), 4437-4440). Weiterhin wurde dort gezeigt, daß dieses Peptid auch nach Zyklisierung durch Verbrückung der beiden Cysteinreste an den Positionen 12 und 32 eine Affinität für den uPA Rezeptor zeigt. In einem alternativen Ansatz wurden von Goodson et al., (Proc. Natl. Acad. USA 91 (1994), 7129-7133) antagonistische uPA-Peptide für den uPAR mittels Musterung einer Bakteriophagen-Peptidbibliothek identifiziert. Diese Peptide zeigten keine ersichtliche Sequenzhomologie zu der natürlichen uPAR-bindenden Sequenz von uPA.

In neueren Veröffentlichungen (Rettenberger et al., Biol. Chem. Hoppe-Seyler 376 (1995), 587-594); Magdolen et al., Eur. J. Biochem. 237 (1996), 743-751; Goretzki et al., Fibrinolysis and Proteolysis 11 (1997), 11-19) sind weitere Untersuchungen an der uPAR-Bindungsregion des uPA beschrieben. Dabei wurden die Reste Cys19, Lys23, Tyr24, Phe25, Ile28, Trp30 und Cys31 als wichtige Determinanten für eine uPA/uPAR-Wechselwirkung identifiziert. Als wirksamster Inhibitor wurde in diesen Untersuchungen ein uPA-Peptid mit den Aminosäuren 16 bis 32 von uPA identifiziert.

Magdolen et al. (1996) supra, analysieren die uPAR-Bindungsregion des uPA-Moleküls unter der Verwendung eines Peptids mit den Aminosäuren 14 bis 32 von uPA und davon abgeleiteten Peptiden. Diese sowie auch die von anderen Arbeitsgruppen verwendeten Peptide (vgl. z.B. Appella et al. (1987) supra) weisen jedoch eine relativ geringe Affinität zum uPAR auf.

WO-A-94/22646 offenbart lineare Peptide mit einer Länge von 6 bis 18 Aminosäuren, die aus dem Bereich der Aminosäuren 14 bis 33 von uPA stammen. Es wird beschrieben, daß kurze von uPA abgeleitete Peptide (uPA 21-29 und uPA 21-26) in der Lage sind, das Wachstum von Keratinozyten zu beeinflussen. Zwar wird in WO-A-94/22646 auf einen möglichen Einsatz der beanspruchten Peptide für die Blockierung der uPA/uPAR-Wechselwirkung hingewiesen, es werden jedoch keinerlei Daten oder Hinweise auf derartige Bindungsstudien gezeigt. Darüber hinaus enthalten die als bevorzugt genannten linearen Peptide uPA 21-29 und uPA 21-26 nicht die minimale uPAR-Bindungsregion von linearen uPA-Peptiden, welche den Sequenzbereich der Aminosäuren 19 bis 31 umfaßt. Die Beeinflussung des Wachstums von Keratinozyten durch diese kurzen Peptide beruht somit sehr wahrscheinlich nicht auf einer uPA/uPAR-Wechselwirkung.

WO 98/46632 offenbart uPAR-Peptidinhibitoren, die vom linearen Peptid uPA (19-31) und zyklischen Derivaten davon abgeleitet sind und an ausgewählten Positionen D-Aminosäurereste tragen.

Ein Beispiel für einen solchen-Peptidinhibitor ist das Peptid cyclo[21,29][D-Cys²¹,Cys²⁹]uPA₂₁₋₃₀. Dieses Peptid besitzt eine bereits recht hohe Affinität für uPAR (IC₅₀ = 78nM), die nur noch um einen Faktor 4 geringer als die Affinität des aminoterminalen Fragments von uPA (ATF = Aminosäuren 1-135 der Urokinase) mit einem IC₅₀-Wert von 21 nM ist. Das entsprechende ausschließlich aus L-Aminosäuren zusammengesetzte Peptid cyclo[(21,29]-[Cys²¹,Cys²⁹]-uPA₂₁₋₃₀ hat eine hundertfach geringe Aktivität im Vergleich zu ATF.

Die der vorliegenden Erfindung zugrundeliegende Aufgabe bestand darin, durch Einbau isostruktureller oder/und isofunktioneller natürlicher bzw. nichtnatürlicher Aminosäuren die Struktur des uPAR-Peptidinhibitors zu modifizieren und somit eine weitere Verbesserung hinsichtlich der Affinität für uPAR, der Serumstabilität oder/und der therapeutischen Wirkung zu erreichen.

Ein Gegenstand der vorliegenden Erfindung sind somit Verbindungen der allgemeinen Strukturformel (I): 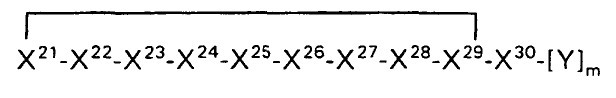 worin
- X²¹-X³⁰: monomere Bausteine, vorzugsweise Aminocarbonsäurereste bedeuten und von einer Struktur mit der Bedeutung X²¹ = D-Cys, X²² = Asn, X²³ = Lys, X²⁴ = Tyr, X²⁵ = Phe, X²⁶ = Ser, X²⁷ = Asn, X²⁸ = Ile, X²⁹ = Cys und X³⁰ = Trp abgeleitet sind,
- Y: ein Spacer ist und m 0 oder 1 ist, und
die monomeren Bausteine über -CONR¹- oder -NR¹CO-Bindungen verknüpft sind, wobei R¹ jeweils unabhängig Wasserstoff, Methyl oder Ethyl bedeutet, und pharmazeutisch verträgliche Salze und Derivate davon,
mit der Maßgabe, daß mindestens einer der Aminosäurereste X²¹-X²⁹ der Leitstruktur durch einen der im folgenden angegebene Aminosäurereste ersetzt ist:
- X²¹:: Asp, Glu, 2,3-Diaminopropionsäure (Dap), 2,4-Diaminobuttersäure (Dab), D-Penicillanin (D-Pen), Allylglycin (Alg), Ornithin (Orn), Lys;
- X²²:: Asp, Glu;
- X²³:: Dab, Dap, His, Citrullin (Cit), Homocitrullin (Hci), Norleucin (Nle);
- X²⁴:: Homophenylaianin (Hph), 1,2,3,4-Tetrahydroisochinolin-3-carbonsäure (Tic), Thienylalanin (Thi), Trp, Phenylglycin (Phg), 1-Naphthylalanin (1-Nal), 2-Naphthylalanin (2-Nal), Cyclohexylalanin (Cha);
- X²⁵:: Trp, Tic, Thi, Hph, Phg;
- X²⁶:: Val;
- X²⁷:: Asp, Glu;
- X²⁸:: Cha, 2-Aminobuttersäure (Abu), tert.-Leucin (Tle), α-Aminoisobuttersäure (Aib);
- X²⁹:: Asp, Glu, Dap, Dab, Alg, D-Pen, Orn, Lys.

Bevorzugt sind Peptide, bei denen mindestens einer der monomeren Bausteine X²¹, X²³, X²⁴, X²⁵, X²⁷ und X²⁸ der Leitstruktur eine der im folgenden angegebenen Bedeutungen besitzt:
- X²¹:: D-Pen;
- X²³:: Dap, Dab, Cit, Hci, Nle, His;
- X²⁴:: Thi, Hph, Phg, 1-Nal, 2-Nal, Cha;
- X²⁵:: Thi;
- X²⁷:: Asp;
- X²⁸:: Val, Cha.

Besonders bevorzugt sind Peptide, in denen mindestens einer der monomeren Bausteine X²¹, X²³, X²⁴, X²⁵ und X²⁸ der Leitstruktur eine der im folgenden angegebenen Bedeutungen besitzt:
- X²¹:: D-Pen;
- X²³:: Dab, Nle, Cit, Hci;
- X²⁴:: 1-Nal, 2-Nal, Cha;
- X²⁵:: Thi;
- X²⁸:: Cha.

Y ist eine Spacergruppe, z. B eine aus einer oder mehreren Aminosäuren aufgebaute peptidische Spacergruppe, z.B. Poly-Lys, oder eine andere Spacergruppe, z.B. eine Polyethylenglykolgruppe. Das Peptid kann über die Gruppe Y an Trägersubstanzen gekoppelt werden.

Die erfindungsgemäßen Peptide sind zyklische Peptide mit einem neungliedrigen Ring, wobei mindestens 2, vorzugsweise mindestens 3 und besonders bevorzugt mindestens 4 der den Ring bildenden Aminosäurereste eine Sequenz aus der uPA-Region 22 bis 28 aufweisen.

Ein weiterer Gegenstand der vorliegenden Erfindung sind Verbindungen der allgemeinen Strukturformel (I): 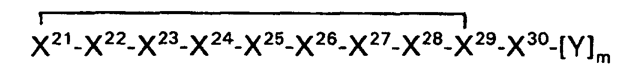 worin
- X²¹-X³⁰: monomere Bausteine, vorzugsweise Aminocarbonsäurereste bedeuten und von einer Struktur mit der Bedeutung X²¹ = D-Cys, X²² = Asn, X²³ = Lys, X²⁴ = Tyr, X²⁵ = Phe, X²⁶ = Ser, X²⁷ = Asn, X²⁸ = Ile, X²⁹ = Cys und X³⁰ = Trp abgeleitet sind,
- Y: ein Spacer ist und m 0 oder 1 ist, und
die monomeren Bausteine über -CONR¹- oder -NR¹CO-Bindungen verknüpft sind, wobei R¹ jeweils unabhängig Wasserstoff, Methyl oder Ethyl bedeutet, und pharmazeutisch verträgliche Salze und Derivate dav.on,
mit der Maßgabe, daß mindestens einer der Aminosäurereste X²¹-X³⁰ der Leitstruktur durch einen nicht-proteinogenen Aminosäurerest ersetzt ist, wobei die resultierenden Verbindungen vorzugsweise eine erhöhte Proteasestabilität, insbesondere eine erhöhte Stabilität gegenüber physiologischen Proteasen, z.B. im Blut oder Gewebe vorkommenden Proteasen, wie etwa Plasmin oder/und im Verdauungstrakt vorkommenden Proteasen wie etwa Pepsin, Trypsin oder Chymotrypsin im Vergleich zur Leitstruktur aufweisen. Vorzugsweise ist zumindest der Aminosäurerest Lys²³ durch eine nicht-proteinogene Aminosäure, d.h. durch eine nicht genetisch kodierte Aminosäure wie etwa Orn, Dap, Dab, Cit, Hci oder Nle ersetzt.

Neben Peptiden der Strukturformel (I) sind auch pharmazeutisch verträgliche Salze und Derivate davon als uPAR-Antagonisten geeignet. Als Derivate kommen dabei insbesondere solche Verbindungen in Betracht, die an reaktiven Gruppen der Seitenkette oder/und des N- bzw. C-Terminus, z. B. an Amino- oder Carbonsäuregruppen, modifiziert sind. Beispiele für solche Modifikationen sind eine Acylierung, z. B. eine Acetylierung von Aminogruppen oder/und eine Amidierung oder Veresterung von Carbonsäuregruppen, z.B. eine Amidierung der C-terminalen Aminosäure.

Die monomeren Bausteine sind über Säureamidbindungen NR¹CO oder CONR¹ verknüpft, d. h. die Peptidsequenzrichtung kann umgekehrt werden (Retropeptide). R¹ kann wie in nativen Polypeptiden Wasserstoff bedeuten. Andererseits kann R¹ jedoch auch einen Alkylrest, z. B. Methyl oder Ethyl, und insbesondere Methyl bedeuten, da durch eine N-Alkylierung der Amidbindung oftmals eine starke Aktivitätsbeeinflussung bewirkt werden kann (vgl. z. B. Levian-Teitelbaum et al., Biopolymers 28 (1989), 51-64). Als monomere Bausteine werden die α-Aminocarbonsäuren - sofern nicht anders angegeben - in Form von L-Enantiomeren eingesetzt.

Die erfindungsgemäßen Peptide sind zyklische Verbindungen, wobei die monomeren Bausteine X²¹ und X²⁹ miteinander verbrückt sind. Diese Verbrückung kann beispielsweise über die Seitenketten der jeweiligen α-Aminocarbonsäurereste erfolgen, wobei eine Verbrückung über Disulfidbindungen, z. B. zwischen zwei Cysteinresten besonders bevorzugt ist. Andere Arten der Zyklisierung zwischen Aminosäureseitenketten sind jedoch auch möglich, z. B. Amidbindungen zwischen einer Aminosäure mit einer Aminoseitengruppe, z. B. Ornithin oder Lys und einer Aminosäure mit einer Carbonsäureseitengruppe wie etwa Asp oder Glu. Weiterhin kann die Disulfidbrücke auch durch eine Alkylen-Brücke ersetzt werden, um die chemische Stabilität zu erhöhen. Darüber hinaus sind auch Verknüpfungen von einer Aminosäureseitenkette mit dem Peptidrückgrat, z. B. Verknüpfung einer Aminoseitengruppe, z.B. einer ω-Aminoseitengruppe, mit dem C-terminalen Ende bzw. Verknüpfung einer Carbonsäureseitengruppe mit dem N-terminalen Ende möglich. Auch eine Verknüpfung von N- und C-Terminus ist möglich. Die erfindungsgemäßen Peptide sind durch chemische Synthese wie in den Beispielen erläutert erhältlich.

Weiterhin betrifft die vorliegende Erfindung eine pharmazeutische Zusammensetzung, die als Wirkstoff mindestens ein Peptid oder Polypeptid wie zuvor definiert gegebenenfalls zusammen mit pharmazeutisch üblichen Träger, Hilfs- oder Verdünnungsmitteln enthält. Insbesondere werden die erfindungsgemäßen Peptide oder Polypeptide zur Herstellung von uPAR-Antagonisten verwendet, die sich auch zur Bekämpfung von mit der Expression von uPAR assoziierten Krankheiten, insbesondere zur Tumorbekämpfung eignen. Weiterhin können erfindungsgemäße Peptide - ebenso wie die Leitstruktur cyclo[21,29][D-Cys²¹,Cys²⁹]-uPA₂₁₋₃₀ als Inhibitoren der Angiogenese eingesetzt werden.

Ein weiterer Gegenstand der vorliegenden Erfindung ist die Verwendung der erfindungsgemäßen uPAR-Peptidantagonisten zur Herstellung von Targetingvehikeln, z.B. Liposomen, viralen Vektoren etc., für uPAR-exprimierende Zellen. Das Targeting kann für diagnostische Anwendungen zum gesteuerten Transport von Markierungsgruppen, z.B radioaktiven oder nichtradioaktiven Markierungsgruppen erfolgen. Andererseits kann das Targeting für therapeutische Anwendungen, z.B. zum Transport von pharmazeutischen Wirkstoffen, beispielsweise auch zum Transport von Nukleinsäuren für die Gentherapie erfolgen.

Die erfindungsgemäßen pharmazeutischen Zusammensetzungen können in beliebiger Form vorliegen, beispielsweise als Tabletten, als beschichtete Tabletten oder in Form von Lösungen oder Suspensionen in wässrigen oder nichtwässrigen Lösungsmitteln. Die Peptide werden vorzugsweise oral oder parenteral in einer flüssigen oder festen Form verabreicht. Bei Verabreichung in flüssiger Form wird Wasser vorzugsweise als Trägermedium verwendet, das gegebenenfalls Stabilisatoren, Löslichkeitsvermittler oder/und Puffer enthält, die üblicherweise für Injektionslösungen verwendet werden. Solche Zusatzstoffe sind beispielsweise Tartrat- oder Boratpuffer, Ethanol, Dimethylsulfoxid, Komplexierungsmittel wie etwa EDTA, Polymere wie etwa flüssiges Polyethylenoxid, etc.

Bei Verabreichung in fester Form können feste Trägersubstanzen wie etwa Stärke, Lactose, Mannitol, Methylcellulose, Talkum, hochdisperses Siliciumoxid, hochmolekulare Fettsäuren wie etwa Stearinsäure, Gelatine, Agar, Calciumphosphat, Magnesiumstearat, tierische und pflanzliche. Fette oder feste hochmolekulare Polymere wie etwa Polyethylenglykole eingesetzt werden. Weiterhin können die Formulierungen zu oralen Applikation sofern gewünscht auch Aroma- und Süßstoffe enthalten.

Die erfindungsgemäßen therapeutischen Zusammensetzungen können auch in Form von Komplexen vorliegen, z.B. mit Cyclodextrinen wie etwa γ-Cyclodextrin. Die verabreichte Dosis hängt vom Alter, Gesundheitszustand und Gewicht des Patienten, von der Art und der Schwere der Erkrankung, von der Art der Behandlung, von der Häufigkeit der Verabreichung und der Art der gewünschten Wirkung ab. Die tägliche Dosis der aktiven Verbindung ist üblicherweise 0,1 bis 50 mg/Kilogramm Körpergewicht. Normalerweise sind 0,5 bis 40 und vorzugsweise 1,0 bis 20 mg/kg/Tag in einer oder mehreren Dosen ausreichend, um die gewünschten Wirkungen zur erreichen.

Weiterhin soll die Erfindung durch die im Nachfolgenden beschriebenen Beispiele und Figuren erläutert werden.

Es zeigen:
- Figur 1: den Vergleich der Affinität der Peptide cyclo[21,29][Cys^{21,29}]-uPA₂₁₋₃₀ und cyclo[21-29][D-Cys²¹,Cys²⁹]-uPA₂₁₋₃₀ (a) bzw. cyclo[21,29][Cys^{21,29}]uPA₂₁₋₃₀ und dem entsprechenden Peptid-Amid(b)
- Figur 2: IC₅₀-Werte von Modifikationen der Leitstruktur cyclo[21,29][D-Cys²¹,Cys²⁹]uPA₂₁₋₃₀;
- Figur 3: eine schematische Darstellung für bevorzugte Modifikationen der Leitstruktur;
- Figur 4: die Stabilität der Peptide cyclo[19,31]-uPA₁₆₋₃₂, cyclo[21,29][D-Cys²¹,Tic²⁵,Cys²⁹]-uPA₂₁₋₃₀ und cyclo[21,29][D-Cys²¹,Cys²⁹]uPA₂₁₋₃₀ in Humanserum (a) und heparinisiertem Humanblut (b);
- Figur 5: die Plasmin-Resistenz von uPA-Peptiden nach Substitution von Lys²³ durch nicht-proteinogene Aminosäuren.

### Beispiele

### 1. Methoden

### 1.1 Festphasenpeptidsynthese

Lineare Peptide wurden auf einem 2-Chlortritylharz (Barlos et al., Int. J. Pept. Protein Res. 37 (1991), 513 bis 520) oder einem Tritylchlorid-Polystyrolharz unter Verwendung eines Applied Biosystems Modell 431 A Peptidsynthesizers bzw. eines multiplen Peptidsynthesizers Modell Syro II (MultiSynTech) synthetisiert. Unter Anwendung der orthogonalen Fmoc-Strategie (Carpino und Han, J. Org. Chem. 37 (1972), 3404-3409; Fields und Noble, Int. J. Peptide Protein Res. 35 (1990), 161-214) wurden die Aminosäureseitenketten mit den Schutzgruppen Trityl (Asn, Cys, Gln und His), tert.-Butyloxycarbonyl (Lys und Trp), tert.-Butyl (Asp, Glu, Ser, Thr und Tyr), Acetamidomethyl (Cys) und 2,2,5,7,8-Pentamethylchroman-6-sulfonyl oder 2,2,4,6,7-Pentamethyldihydrobenzofuran-5-sulfonyl (Arg) blockiert. Die Kupplung erfolgte bei Raumtemperatur in Dimethylformamid unter Verwendung eines dreifachen Überschusses von 2-(1H-Benzotriazol-1-yl)-1,1,3,3-tetramethyluroniumtetrafluoroborat/1-Hydroxybenzotriazol/Fmoc-Aminosäure mit 2,5 Äquivalenten von N-Ethyldiisopropylamin in N-Methylpyrrolidon. Die Fmoc-Gruppe wurde durch sequenzielle Behandlung der Harze mit einem Überschuß von 40 % bzw. 20 % Piperidin in Dimethylformamid entfernt. Die Abspaltung der Peptide und die Entfernung der Seitenkettenschutzgruppen wurde gleichzeitig durch Behandlung mit 93 % Trifluoressigsäure/5 % Triisopropylsilan/3 % H₂O (0 °C/1 h; Raumtemperatur/1h) durchgeführt. Im Fall von mit 2,2,5,5,7,8-Pentamethylchroman-6-sulfonyl-geschützten Arg-Gruppen wurden die Peptide für zusätzliche 12 h bei Raumtemperatur inkubiert. Die rohen Peptide wurden mit Diethylether bei -30 °C präzipitiert, in Methanol gelöst, wie zuvor präzipitiert, in tert.-Butanol gelöst und lyophilisiert. Tryptophan enthaltende Peptide wurden zusätzlich mit 5 % Essigsäure für 2 h vor der Lyophilisierung behandelt.

Die Peptide wurden durch HPLC unter Verwendung einer Reverse Phase C-18 Säule (Nucleosil 1005-C18) oder einer YMC-Pack ODS Säule gereinigt. Die Zyklisierung erfolgte durch eine Disulfidbrückenbildung. Die hierzu erforderliche Oxidation wurde durch Aufnehmen von 0,1 bis 0,3 mg/ml der gereinigten linearen Peptide in 80 % Wasser und 20 % DMSO (Vol/Vol) und Entfernen des Lösungsmittels unter verringertem Druck nach 10 h durchgeführt. Die zyklischen Peptide wurden erneut durch HPLC wie zuvor beschrieben gereinigt.

### 1.2 Massenspektroskopie und Aminosäureanalyse

Die gereinigten und entsalzten Peptide wurden auf dem HPLC-System 140 B (Applied Biosystems, Foster City, USA) analysiert. Die UV-Absorption wurde mit einem UVIS 200 Detektor (Linear Instruments, Reno, USA) bei 206 nm gemessen. Die Chromatographie erfolgte auf einer Aquapore 3 µ (Applied Biosystems, Foster City, USA) Reverse Phase Säule (1 mm x 50 mm) mit einer Durchflußrate von 20 µl/min. Das Lösungsmittelsystem war 0,1 % TFA in Wasser (A) und 0,1 % TFA in Acetonitril (B). Das HPLC System war an eine Atmosphärendruck-Ionisationsquelle gekoppelt, die an ein Tandem-Quadrupolinstrument API III (Sciex, Perkin Eimer, Thornhill, Kanada) angeschlossen war.

Die Quadrupol M/Z-Skala wurde mit den Ammoniumadduktionen von Polypropylenglykol kalibriert. Die durchschnittlichen Massenwerte wurden von den M/Z-Peaks in den Ladungsverteilungsprofilen der mehrfach geladenen Ionen berechnet (Covey et al., Rapid Commun. Mass Spectrom. 2 (1988), 249-256; Fenn et al., Science 246 (1989), 64-71).

Die Aminosäureanalyse wurde nach der Ninhydrinmethode unter Verwendung des Analysesystems 6300 (Beckman Instruments, Fullerton, USA) nach Hydrolyse der Peptide durch die TFA-HCl-Dampfphasenmethode (Tsugita et al., J. Biochem. 102 (1987), 1593-1597) durchgeführt, die eine quantitative Bestimmung der Peptidkonzentration erlaubt.

### 1.3 Durchflußzytometrie

Die Kapazität der synthetischen Peptide zu Hemmung der uPA/uPAR-Wechselwirkung wurde mittels Durchflußzytometrie an dem FACScan Durchflußzytometer (Becton-Dickinson, Heidelberg, Deutschland) unter Verwendung der humanen Promyeloidzellinie U937 als Quelle für zellulären nativen uPAR bestimmt (Chuchulowski et al., Fibrinolysis 6, Suppl. 4 (1992), 95-102; Magdolen et al., (1996), supra). Die U937 Zellen wurden mit 1 mM Phorbol-12-myristat-13-acetat (PMA) für 48 h stimuliert. Nach Stimulierung mit PMA exprimieren die U937 Zellen beträchtliche Mengen an zelloberflächenassoziiertem uPAR.

Die stimulierten Zellen wurden mit 50 mM Glycin-HCl, 0,1 NaCl, pH 3,6 für 1 min bei Raumtemperatur behandelt, um endogenen rezeptorgebundenen uPA zu dissoziieren. Anschließend wurde der saure Puffer durch 0,5 M HEPES-100 mM NaCl, pH 7,5 neutralisiert. Die Zellen wurden dann sofort zweimal mit PBS/0,1 % Rinderserumalbumin (RSA) gewaschen und für 10 min bei Raumtemperatur und 300 x g zentrifugiert. Die Zellen wurden in PBS/0,1 % RSA resuspendiert, auf eine Konzentration von 10⁶ Zellen pro ml eingestellt und simultan mit 16 ng FITC-konjugiertem pro-uPA und unterschiedlichen Mengen der synthetischen Peptide für 45 min bei Raumtemperatur inkubiert. Vor der Analyse wurde Propidiumiodid, ein Fluoreszenzfarbstoff, der spezifisch doppelsträngige DNA bindet, zu jeder Probe gegeben, um die Lebensfähigkeit der analysierten U937 Zellen zu bestimmen. Die beschädigten, Propidiumiodid-markierten Zellen wurden von der Analyse ausgeschlossen.

### 1.4 Festphasen uPAR/uPA-Bindetest

Zusätzlich zu den durchflußzytometrischen Analysen wurde ein Festphasen ATF-Ligandenbindetest zur Untersuchung der Wechselwirkungen von synthetischen Peptiden mit uPAR durchgeführt. Hierzu wurden Mikrotiterplatten mit rekombinantem humanem uPAR aus CHO Zellen (Wilhelm et al., FEBS Lett. 337 (1994), 131-134; Magdolen et al., Elektrophoresis 16 (1995), 813-816) beschichtet und die restlichen proteinbindenden Stellen mit 2 % RSA (Gew/Vol) abgesättigt. Nach Inkubation mit den Proben (0,6 ng ATF zusammen mit 15 µg synthetisches Peptid pro ml) und mehrfachen Waschschritten wurde die Menge an ATF, die an den auf der Mikrotiterplatte immobilisierten uPAR gebunden hatte, unter Verwendung eines biotinylierten monoklonalen Mausantikörpers gegen die Kringeldomäne von ATF (Nr. 377, American Diagnostica, Greenwich, CT, USA) und anschließender Zugabe von Avidin-Peroxidase-Konjugat und 3,3',5,5'-Tetramethylbenzidin/H₂O₂ als Substrat für die Peroxidase bestimmt. Das Vorhandensein von synthetischen Peptiden, die mit der ATF-Bindung an uPAR kompetieren, verringert den Umsatz des chromogenen Substrats.

### 1.5 Bestimmung der Stabilität von Peptiden in Körperflüssigkeiten

Die Stabilität von uPA-abgeleiteten Peptiden in humanem Serum oder Vollblut wurde in vitro getestet.

Für die Präparation von Humanseren wurde venöses Blut ohne Antikoagulans in Polypropylen-Röhrchen bei 37°C 45 min gerinnen gelassen. An der Gefäßwand anhaftende Gerinnsel wurden mit einem Plastikstäbchen abgelöst und bei 1200 x g 1 2 min bei Raumtemperatur abzentrifugiert. Das überstehende Serum wurde abgenommen und entweder frisch für Stabilitätsuntersuchungen eingesetzt oder aliquotiert bei -20°C zur späteren Verwendung eingefroren.

Für Stabilitätsuntersuchungen in humanem Vollblut wurde venöses Blut durch Heparin-Natrium (1000 I.E. je 10 ml Vollblut) gerinnungsgehemmt und frisch verwendet. Die zu prüfenden Peptide wurden in Form von Peptidgemisch-Stammlösungen (≥ 1 µg je Peptid in H₂O) den Körperflüssigkeiten in einer Konzentration von 5 µg/100 µl Serum bzw. 200 µl Vollblut (je Peptid) zugefügt und für verschiedene Zeiten bei 37°C inkubiert. Vor der Analyse durch HPLC wurden Vollblutinkubationen 5 min bei Raumtemperatur 16.000 x g zentrifugiert und das überstehende Plasma abgenommen.

Seren und Plasmen wurden vor der HPLC-Analytik über HLB-Vorsäulen (Waters GmbH, Oasis HLB extraction cartridge 1 cm³/30 mg) vorgereinigt. Dazu wurden 100 µl der Flüssigkeiten mit PBS auf 1 ml verdünnt und auf die mit 1 ml 100% Methanol und 1 ml H₂O konditionierte HLB-Vorsäulen aufgetragen. Die Säulen wurden mit 1 ml 5% Methanol in H₂O gewaschen und mit 1 ml 100% Methanol eluiert. Von dem Eluat wurden die ersten 200 µl (4 Tropfen) als Totvolumen verworfen. Die folgenden 500 µl Eluat wurden mit 500 µl PBS verdünnt und HPLC-analysiert. Die restlichen 300 µl des Eluats wurden verworfen. Die HPLC-Analytik wurde durchgeführt mit einer YMC-5 C₁₈ analytischen Säule und einem 20-60% Gradienten aus H₂O, 0,1 % Trifluoressigsäure und Acetonitril, 0,1 % Trifluoressigsäure über 30 min und Detektion der Analyten bei 220 nm.

### 1.6 Bestimmung der Protease-Resistenz

Die Empfindlichkeit von Peptiden gegen den Angriff verschiedener Proteasen des Verdauungstrakts wurde mit gereinigten Enzymen unter geeigneten Pufferbedingungen in vitro durchgeführt. Generell wurden 10 µg Peptid in einem Volumen von 100 µl 30 min bei 37°C mit 2 µg Protease unter den vom Hersteller für die Aktivitätskontrollen angegebenen Pufferbedingungen inkubiert. Nach Inkubation wurden die Ansätze nach Verdünnung mit 300 µl H₂O ohne Vorreinigung über eine Vorsäule direkt durch HPLC mit einem 20-60% Gradienten aus H₂O, 0,1 % Trifluoressigsäure und Acetonitril, 0, 1 % Trifluoressigsäure analysiert.

Pepsin (Sigma, Deisenhofen, Deutschland) wurde mit Peptiden in 52 mM HCl inkubiert. Inkubationen mit Trypsin (Sigma) wurde in 63 mM Natriumphosphat pH 7,6 durchgeführt. Chymotrypsine α, β, γ, δ (ICN) wurden in 50 mM CaCl₂ und 40 mM Tris/Cl pH 7,8 inkubiert. Mit bakterieller Proteinase K wurde in ungepuffertem Wasser inkubiert. Als Positivkontrolle wurde das Peptid cyclo[19,31]-uPA₁₆₋₃₂, das mit Ausnahme der Cysteinverbrückung der Originalsequenz des uPA-Omega-Loops entspricht, eingesetzt. Die Empfindlichkeit von Peptiden gegen die Gewebeprotease Plasmin (Sigma) wurde in Ansätzen mit 10 oder 5 µg Peptiden und 0,05 U Plasmin in 100 µl 200 mM Natriumphosphat pH 7,5 bei 37°C und einer Inkubationsdauer von 30 min getestet.

### 2. Ergebnisse

### 2.1 Inhibitorische Wirkung des Peptids cyclo[21,29] [D-Cys²¹,Cys²⁹]uPA₂₁₋₃₀

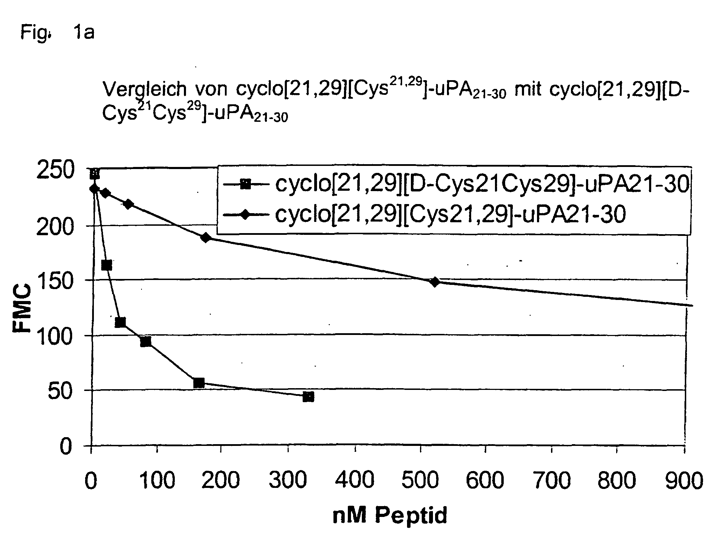

Figur 1a zeigt die inhibitorische Wirkung des Peptids cyclo[21,29] [D-Cys²¹,Cys²⁹]uPA₂₁₋₃₀ im Vergleich zu dem ausschließlich aus L-Aminosäuren bestehenden zyklischen Peptid cyclo[21,29][Cys^{21,29}]-uPA₂₁₋₃₀. Der IC₅₀ Wert des zyklischen Peptids mit D-Cys an Position 21 wurde mit 78 nM bestimmt, während der IC₅₀ Wert des nur aus L-Aminosäuren zusammengesetzten zyklischen Peptids mit 2260 nM bestimmt wurde. Der IC₅₀ Wert des aminoterminalen Fragments von uPA (Aminosäuren 1 bis 135 von uPA) liegt im Vergleich dazu bei 21 nM.

### 2.2 Synthese von modifizierten uPA Peptiden

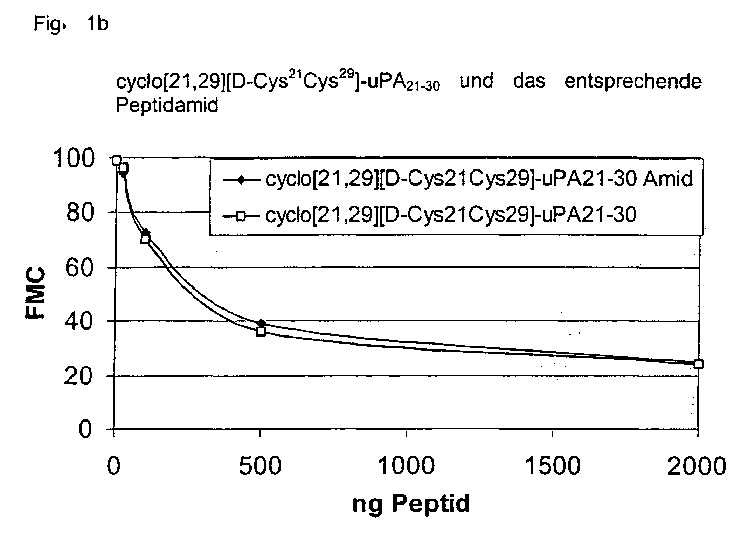

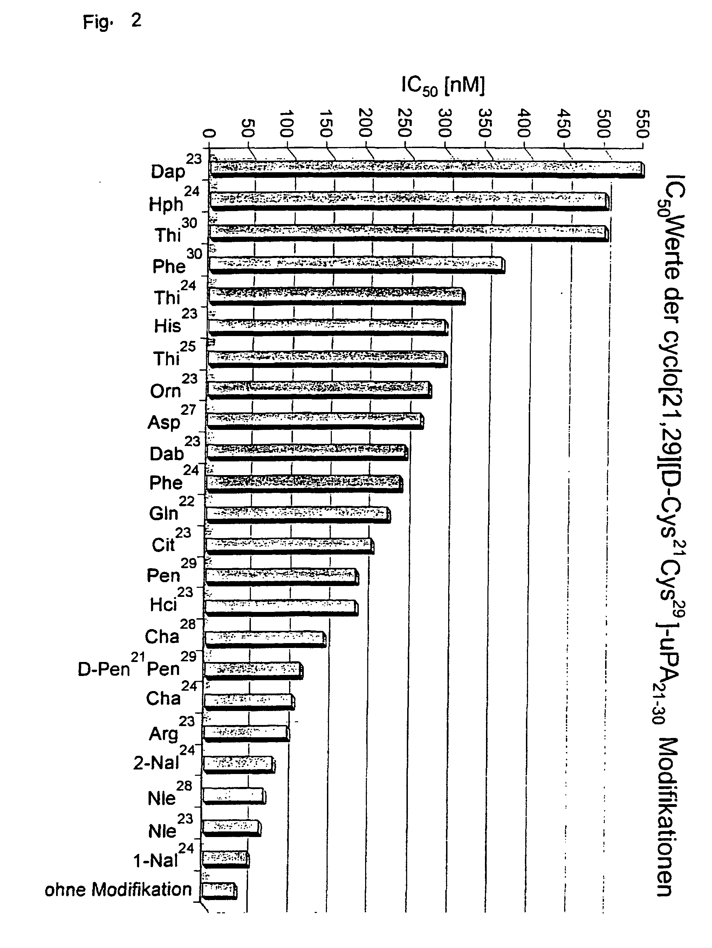

Unter Verwendung von cyclo[21,29][D-Cys²¹,Cys²⁹]uPA₂₁₋₃₀ als Leitstruktur wurden weitere zyklische Peptide hergestellt, in denen bestimmte Aminosäuren durch andere, insbesondere nicht-proteinogene Aminosäuren substituiert wurden. Die relativen Aktivitäten im Vergleich zur Leitstruktur sind in Figuren 1b und 2 gezeigt.

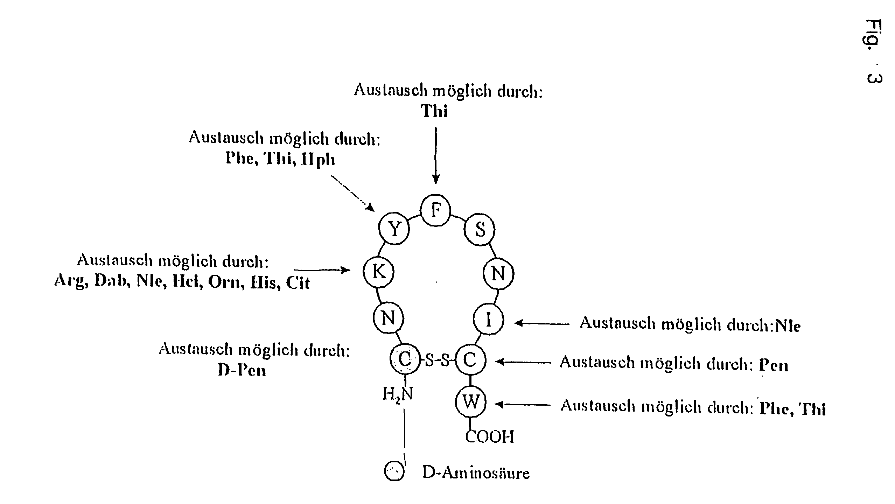

Figur 3 zeigt Beispiele für besonders bevorzugte Modifikationen der Leitstruktur.

### 2.3 Ergebnisse der Untersuchungen zur Stabilität von Peptiden in humanem Serum und Vollblut

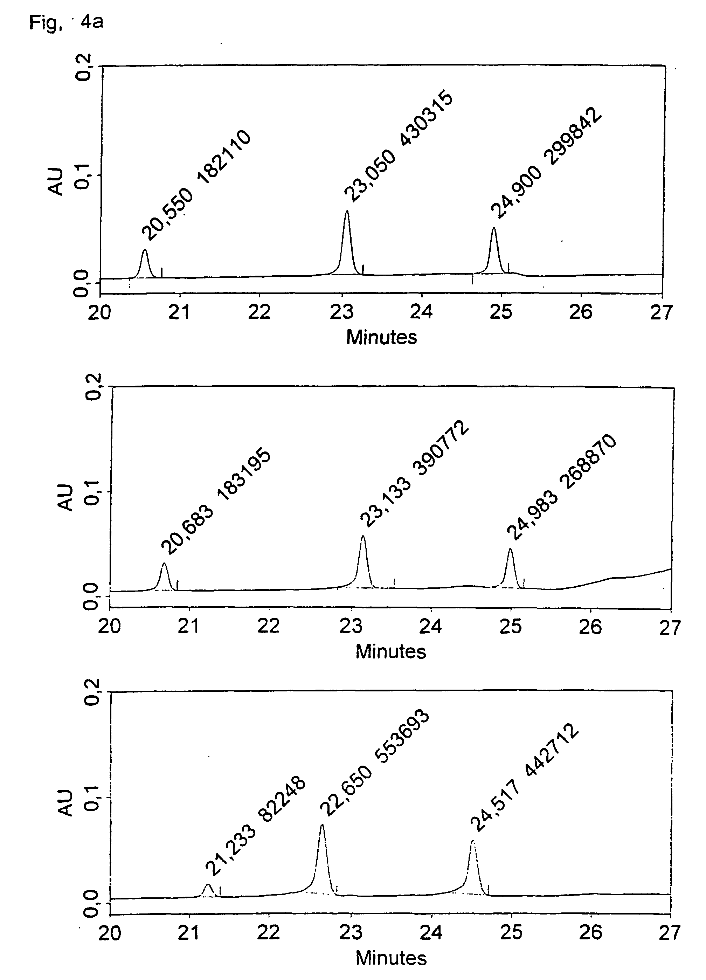

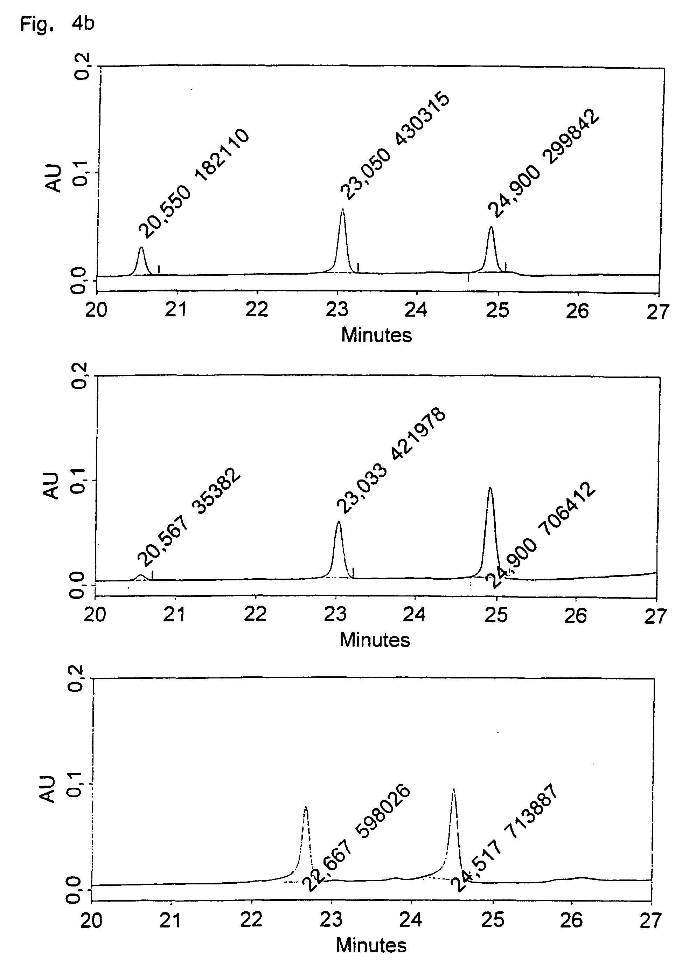

Es wurde die Stabilität der Peptide cyclo[19,31]-uPA₁₆₋₃₂ (A), cyclo[21,29][D-Cys²¹,Tic²⁵,Cys²⁹]-uPA₂₁₋₃₀ (B) und cyclo[21,29][D-Cys²¹,Cys²⁹]-uPA₂₁₋₃₀ (C) in Humanserum und heparinisiertem Humanblut getestet. Die Ergebnisse dieser Versuche sind in Figur 4a und 4b gezeigt. Die Peaks bei 20,5 min entsprechen dem Peptid A, die Peaks bei 23,0 min entsprechen dem Peptid B und die Peaks bei 24,9 min entsprechen dem Peptid C.

Zur Untersuchung der Stabilität der Peptide in humanem Serum (Fig. 4a) wurde ein Gemisch von jeweils 12,5 µg der Peptide zu rund 250 µl Serum gegeben. 100 µl davon wurden mit PBS auf 1 ml verdünnt, auf einer HLB-Vorsäule vorgereinigt und sofort durch HPLC analysiert (mittleres Profil). Weitere 100 µl wurden nach 20,5 h Inkubation bei 37°C (unteres Profil) analysiert. Als Kontrolle wurden jeweils 5 µg der Peptide mit 1 ml PBS versetzt und analysiert (oberes Profil). Der Peak bei 21,2 min entspricht einem nicht identifizierten Metaboliten.

Zur Untersuchung der Stabilität in heparinisiertem Humanblut (Figur 4b) wurden 37,5 µg der Peptide-mit 750 µl frisch präpariertem heparinisiertem Humanblut versetzt. 375 µl wurden sofort danach abzentrifugiert. 100 µl des Plasmaüberstands wurden analysiert (mittleres Profil). Die restlichen 375 µl wurden für 20, 5 h bei 37°C unter leichtem Schütteln inkubiert und anschließend analysiert (unteres Profil). Als Kontrolle wurden bereits 5 µg der Peptide mit 1 ml PBS versetzt und analysiert (oberes Profil).

Nach 20,5 h Inkubation in humanem Serum bei 37°C konnte das Peptid A (cyclo[19,31]-uPA₁₆₋₃₂) nicht mehr nachgewiesen werden. Statt dessen erschien ein neuer Peak mit relativ verlängerter Retentionszeit (Abb. 4a unterstes HPLC-Profil, RT 21,233 min), der vermutlich einem Metaboliten von A entspricht. Die relativen Retentionszeiten und Peak-Integrale der Peptide B (cyclo[21,29][D-Cys²¹Tic²⁵Cys²⁹]-uPA₂₁₋₃₀) und C(cyclo[21,29][D-Cys²¹Cys²⁹]-uPA₂₁₋₃₀) blieben dagegen nach Exposition gegen Humanserum annähernd gleich. Daraus kann auf eine unveränderte chemische Identität und Konzentration der beiden Peptide nach 20,5-stündiger Exposition gegen Humanserum geschlossen werden.

Auch die Exposition der Peptide gegen frisch isoliertes heparinisiertes Vollblut über 20,5 h zeigte die Instabilität von A. In diesem Fall konnte weder die unveränderte Substanz noch ein vermuteter Metabolit detektiert werden. Demgegenüber erschienen die Peaks der Peptide B und C im Humanblut stabil. Der Peak von A erscheint im Vergleich mit der PBS-Kontrolle bereits in der unmittelbar nach Zugabe zum Vollblut aufgearbeiteten und analysierten Probe wesentlich reduziert. Offenbar genügte die kurze Zeitspanne zwischen Zugabe der Peptide und Vorreinigung auf Vorsäulen (10-15 min) aus, einen wesentlichen Teil der zugefügten Peptidmenge (>80%) abzubauen.

Die Peak-Integrale der beiden D-Cys-Derivate B und C aus Plasma sind deutlich größer als in der PBS-Kontrolle, obwohl sie bezogen auf die Gesamtvolumina von PBS und Vollblut in gleicher Konzentration eingesetzt wurden. Nach Inkubation wurden die Peptide aber nur im Plasma nach Entfernung der Blutkörperchen analysiert. Dies kann als Hinweis gewertet werden, daß die D-Cys-Derivate sich vorwiegend im Plasma verteilen, aber nicht signifikant in Biutzellen eindringen können bzw. binden können.

### 2.4 Stabilität von uPA-Peptiden gegen Plasmin

Geprüft auf die Empfindlichkeit gegen Angriff durch die Gewebeprotease Plasmin wurden die Peptidleitstruktur cyclo[21,29][D-Cys²¹Cys²⁹]-uPA₂₁₋₃₀ und deren Modifikationen an der Position 23 cyclo[21,29][D-Cys²¹-Orn²³Cys²⁹]-uPA₂₁₋₃₀(Ornithin),cyclo[21,29][D-Cys²¹Dab²³Cys²⁹]-uPA₂₁₋₃₀(2,4-Diaminobuttersäure),cyclo[21,29][D-Cys²¹Dap²³Cys²⁹]-uPA₂₁₋₃₀(2,3-Diaminopropionsäure), cyclo[21,29][D-Cys²¹Nle²³Cys²⁹]-uPA₂₁₋₃₀ (Norleucin) und cyclo[21,29][D-Cys²¹Arg²³Cys²⁹]-uPA₂₁₋₃₀ (Arginin). Die Leitstruktur cyclo[21,29][D-Cys²¹Cys²⁹]-uPA₂₁₋₃₀ enthält die aus Urokinase bekannte Plasminspaltstelle, nämlich die Peptidbindung zwischen Lys²³ und Tyr²⁴.

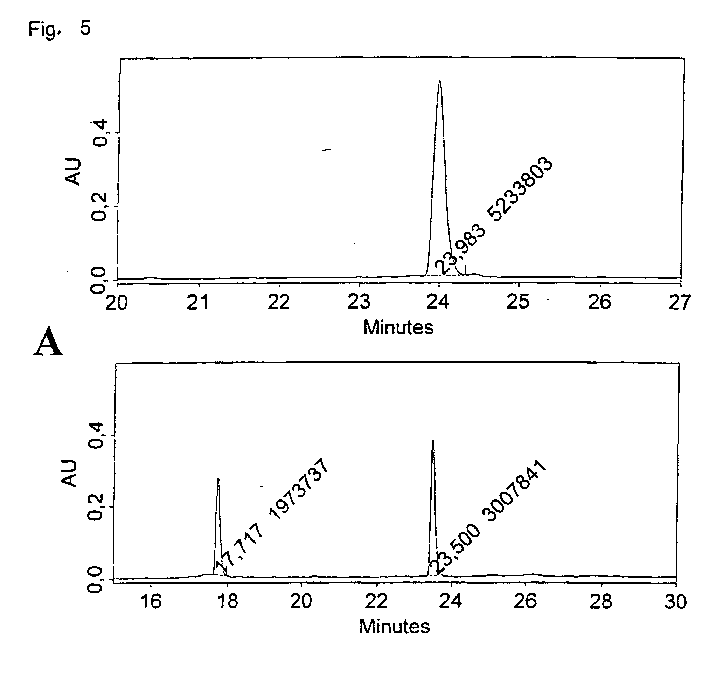

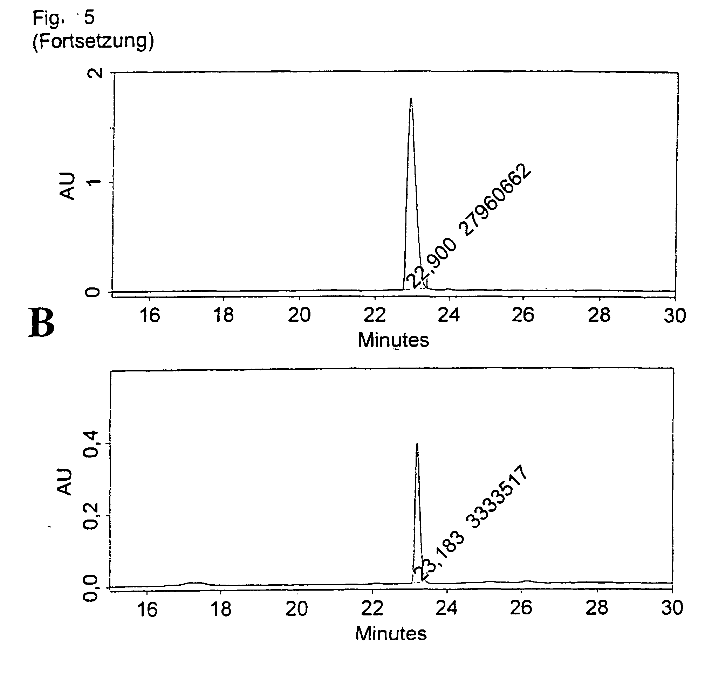

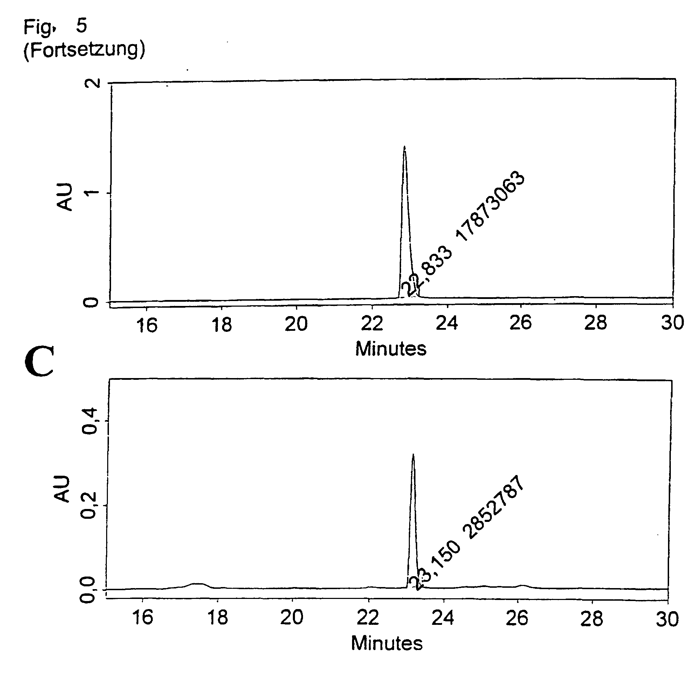

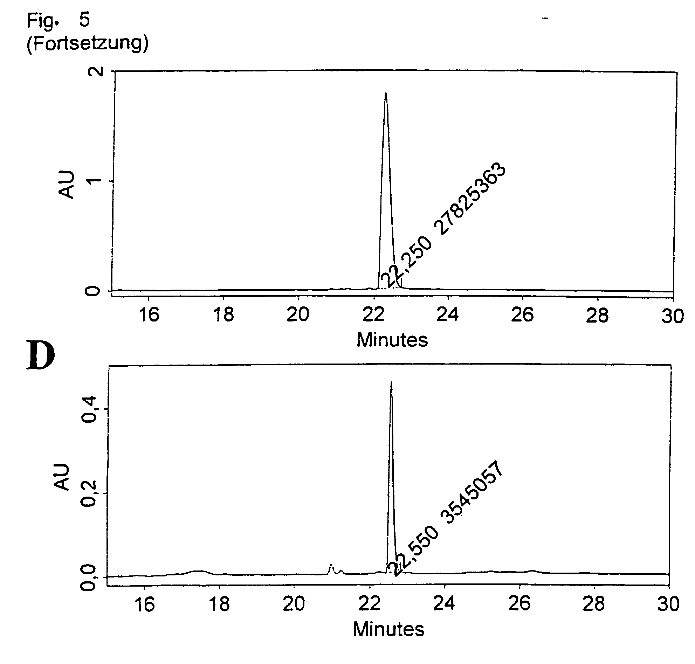

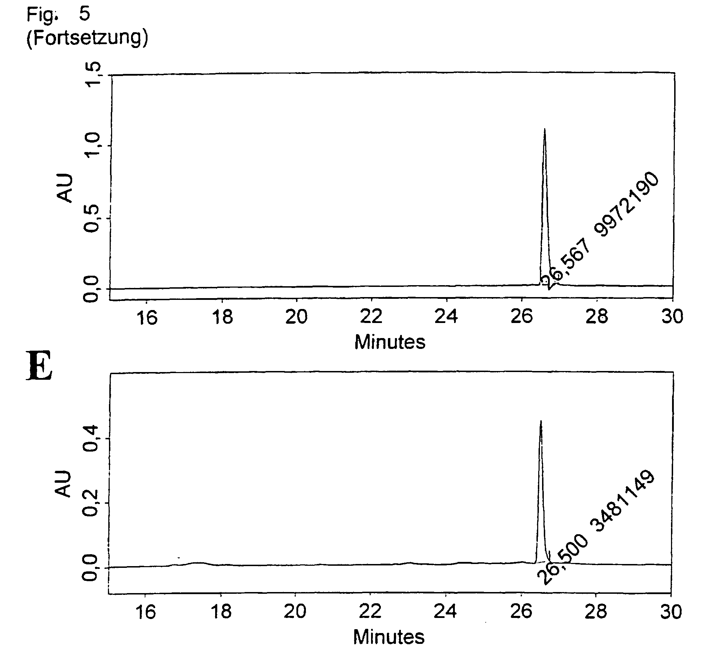

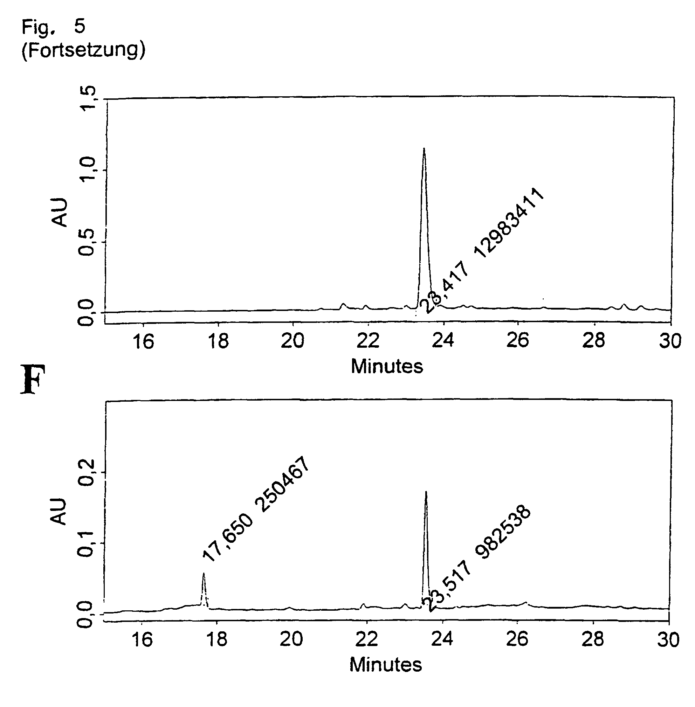

Figur 5 zeigt HPLC-Profile von Peptidvarianten vor (obere Profile) und nach (untere Profile) Inkubation mit Plasmin. Teilabbildung (A) zeigt die unveränderte Leistruktur cyclo[21,29][D-Cys²¹Cys²⁹]-uPA₂₁₋₃₀ mit Lysin an Position 23, (B) die Ornithin-substituierte Variante cyclo[21,29][D-Cys²¹Orn²³Cys²⁹]-uPA₂₁₋₃₀, (C) die Diaminobuttersäure-substituierte Variante cyclo[21,29][D-Cys²¹Dab²³Cys²⁹]-uPA₂₁₋₃₀, (D) die Diaminopropionsäure-substituierte Variante cyclo[21,29][D-Cys²¹Dap²³Cys²⁹]-uPA₂₁₋₃₀, (E) die Norleucin-substituierte Variante cyclo[21,29][D-Cys²¹Nle²³Cys²⁹]-uPA₂₁₋₃₀ und (F) die Argininsubstituierte Varinate cyclo[21,29][D-Cys²¹Arg²³Cys²⁹]-uPA₂₁₋₃₀. Die Protease Plasmin erscheint in den unteren Profilen jeweils bei ca. 17,5 min.

Nach Inkubation von cyclo[21,29][D-Cys²¹Cys²⁹]-uPA₂₁₋₃₀ mit Plasmin und HPLC-Analyse der Produkte trat neben dem Peak der unveränderten Leitstruktur ein neuer, unbekannter Peak auf. Die Summe beider Peak-Integrale entsprach 93,5 % des Peak-Integrals der unveränderten Leitstruktur. Bei dem neuen Peak handelte es sich demnach mit hoher Wahrscheinlichkeit um das Plasmin-Spaltprodukt von cyclo[21,29][D-Cys²¹Cys²⁹]-uPA₂₁₋₃₀. Von den an Position 23 modifizierten Peptiden, die alle in Bezug auf die Kompetition mit uPA um die Bindung an den uPAR aktiv waren, erwiesen sich die Diaminbuttersäure-, Ornithin- und Norleucinsubsitutierten als stabil gegen Plasmin (Figur 5). Bei der Arg²³-substituierten Variante trat nach Inkubation mit Plasmin ein neuer Peak auf, dessen Retentionszeit mit derjenigen des Plasminmetaboliten der unveränderten Leitstruktur annähernd identisch war, d.h. die Arg²³-substituierte Variante ist Plasmin-sensibel. Bei der Plasmin-Exposition der Diaminpropionsäuresubstituierten Variante traten zwei kleine unidentifizierte Peaks bei ca. 21 min auf. Deren Retentionszeit unterscheidet sich stark von den Retentionszeiten der Plasmin-Metaboliten der Leitstruktur und derjenigen der Arg²³-Variante. Entsprechend fraglich ist, ob die kleinen 21 min-Peaks tatsächlich spezifische Plasminspaltprodukte der Dap²³-Variante darstellen.

Durch Substitution von Lysin an Position 23 der cyclo[21,29][D-Cys²¹Cys²⁹]-uPA₂₁₋₃₀ Leitstruktur durch nicht-proteinogene Aminosäuren kann Stabilität gegen die Gewebeprotease Plasmin erzeugt werden, ohne die biologische Aktivität wesentlich zu verändern.

### 2.5 Antiangiogenetische Wirksamkeit

Thorax-Aorten wurden aus 1 bis 2 Monate alten Wistar-Ratten gewonnen und sofort in eine Kulturschale mit serumfreiem Medium (RPMI) überführt. Das Gewebe um die Aorta wurde sorgfältig entfernt. Es wurden 1 mm lange Aortenringe präpariert und gründlich mit serumfreiem Medium gewaschen.

Vor dem Einbetten der Aortenringe in Matrigel wurde der Boden jeder Vertiefung mit 80 µl Gellösung beschichtet. Nach Gelbildung wurden die Aortenringe in die Vertiefung überführt, positioniert und durch Überschichten mit 70 µl Gellösung befestigt. Nach Gelbildung wurden verschiedene Mengen des jeweiligen Testpeptids in die Vertiefungen gegeben. Als Kontrollen wurden Medium alleine, Medium mit Wachstumszusätzen und Medium mit Wachstumszusätzen und Kontrollpeptid untersucht. Die Kulturen wurden bei 35°C für 5 Tage gehalten und anschließend untersucht.

Die Testpeptide waren cyclo[21,29][D-Cys²¹,Cys²⁹]uPA₂₁₋₃₀ und cyclo[21,29][D-Cys²¹,Nle^{23,}Cys²⁹]-uPA₂₁₋₃₀ in Dosierungen von 0,001, 0,1, 1, 10, 25 und 50 µg/ml. Die Testpeptide zeigten deutliche antiangiogenetische Wirkungen im in vitro Gewebekulturtest. Eine Hemmung der Kapillarbildung wurde im Konzentrationsbereich von etwa 1 µg/ml und höher gefunden. Das Sprossen von Kapillaren, d.h. die Anzahl und Länge neu gebildeter Kapillaren konnte im Vergleich mit unwirksamen Kontrollpeptiden bei einer Peptidkonzentration von 30 µg/ml um etwa den Faktor 3 bis 4 verringert werden.

## Patentansprüche

1. Verbindungen der allgemeinen Strukturformel (I): 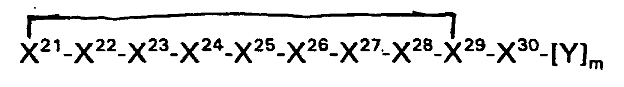 worin X²¹-X³⁰ monomere Bausteine, vorzugsweise Aminocarbonsäurereste bedeuten und von einer Struktur mit der Bedeutung X²¹ = D-Cys, X²² = Asn, X²³ = Lys, X²⁴ = Tyr, X²⁵ = Phe, X²⁶ = Ser, X²⁷ = Asn, X²⁸ = Ile, X²⁹ = Cys und X³⁰ = Trp abgeleitet sind, Y ein Spacer ist und m 0 oder 1, und die monomeren Bausteine über -CONR¹- oder -NR¹CO-Bindungen verknüpft sind, wobei R¹ jeweils unabhängig Wasserstoff, Methyl oder Ethyl bedeutet, und pharmazeutisch verträgliche Salze und Derivate davon,
ist mit der Maßgabe daß mindestens einer der monomeren Bausteine X²¹-X²⁹ der Leitstruktur durch einen der im folgenden angegebenen Aminosäurereste ersetzt ist:
X²¹: Asp, Glu, 2,3-Diaminopropionsäure (Dap), 2,4-Diaminobuttersäure (Dab), D-Penicillanin (D-Pen), Allylglycin (Alg), Ornithin (Orn), Lys;
X²²: Asp, Glu;
X²³: Dab, Dap, His, Citrullin (Cit), Homocitrullin (Hci), Norleucin (Nle);
X²⁴: Homophenylalanin (Hph), 1,2,3,4-Tetrahydroisochinolin-3-carbonsäure (Tic), Thienylalanin (Thi), Trp, Phenylglycin (Phg), 1-Naphthylalanin (1-Nal), 2-Naphthylalanin (2-Nal), Cyclohexylalanin (Cha);
X²⁵: Trp, Tic, Thi, Hph, Phg;
X²⁶: Val;
X²⁷: Asp, Glu;
X²⁸: Cha, 2-Aminobuttersäure (Abu), tert.-Leucin (Tle), α-Aminoisobuttersäure (Aib);
X²⁹: Asp, Glu, Dap, Dab, Alg, D-Pen, Orn, Lys.

2. Verbindungen nach Anspruch 1,
**dadurch gekennzeichnet,**
**daß** mindestens einer der monomeren Bausteine X²¹, X²³, X²⁴, X²⁵, X²⁷ und X²⁸ der Leitstruktur eine der im folgenden angegebenen Bedeutungen besitzt:
X²¹: D-Pen;
X²³: Dap, Dab, Cit, Hci, Nle, His;
X²⁴: Thi, Hph, Phg, 1-Nal, 2-Nal, Cha;
X²⁵: Thi;
X²⁷: Asp;
X²⁸: Val, Cha.

3. Verbindungen nach Anspruch 1,
**dadurch gekennzeichnet,**
**daß** mindestens einer der monomeren Bausteine X²¹, X²³, X²⁴, X²⁵ und X²⁸ der Leitstruktur eine der im folgenden angegebenen Bedeutungen besitzt:
X²¹: D-Pen;
X²³: Dab, Nle, Cit, Hci;
X²⁴: 1-Nal, 2-Nal, Cha;
X²⁵: Thi;
X²⁸: Cha.

4. Verbindungen der allgemeinen Strukturformel (I): 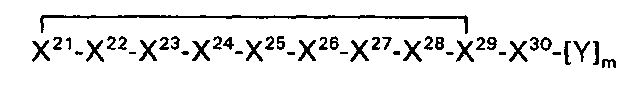 worin X²¹-X³⁰ monomere Bausteine, vorzugsweise Aminocarbonsäurereste bedeuten und von einer Struktur mit der Bedeutung X²¹ = D-Cys, X²² = Asn, X²³ = Dap, Dab oder Nle, X²⁴ = Tyr, X²⁵ = Phe, X²⁶ = Ser, X²⁷ = Asn, X²⁸ = Ile, X²⁹ = Cys und X³⁰ = Trp abgeleitet sind, Y ein Spacer ist und m 0 oder 1, und die monomeren Bausteine über -CONR¹- oder -NR¹CO-Bindungen verknüpft sind, wobei R¹ jeweils unabhängig Wasserstoff, Methyl oder Ethyl bedeutet, und pharmazeutisch verträgliche Salze und Derivate davon.

5. Verbindungen nach einem der vorhergehenden Ansprüche,
**dadurch gekennzeichnet,**
**daß** mindestens 2 der Aminosäurereste X²², X²³, X²⁴, X²⁵, X²⁶, X²⁷, X²⁸ und X³⁰ eine gleiche Seitenkette wie eine Aminosäure an gleicher Position in der nativen uPA-Sequenz aufweisen.

6. Verbindungen nach Anspruch 5,
**dadurch gekennzeichnet,**
**daß** mindestens 2 der Aminosäurereste X²⁴, X²⁵, X²⁸ und X³⁰ die gleiche Seitenkette wie in der nativen uPA-Sequenz aufweisen.

7. Pharmazeutische Zusammensetzung, die als Wirkstoff mindestens eine Verbindung nach einem der Ansprüche 1 bis 6 gegebenenfalls zusammen mit pharmazeutisch üblichen Träger-, Hilfs- oder Verdünnungsmitteln enthält.

8. Verwendung einer Verbindung nach einem der Ansprüche 1 bis 6 zur Herstellung eines uPAR-Antagonisten.

9. Verwendung einer Verbindung nach Anspruch 8 zur Herstellung eines Mittels zur Bekämpfung von mit der Expression von uPAR assoziierten Krankheiten, insbesondere zur Tumorbekämpfung.

10. Verwendung einer Verbindung nach einem der Ansprüche 1 bis 6 zur Herstellung eines Targetingvehikels für uPAR-exprimierende Zellen.

11. Verwendung einer Verbindung nach einem der Ansprüche 1 bis 6 zur Herstellung eines Angiogenese-Inhibitors.

## Claims

1. Compounds of the general structural formula (I): 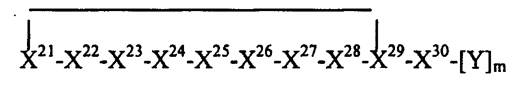 wherein
X²¹-X³⁰ denote monomeric building blocks, preferably aminocarboxylic acid residues and are derived from a structure in which X²¹ = D-Cys, X²² = Asn, X²³ = Lys, X²⁴ = Tyr, X²⁵ = Phe, X²⁶ = Ser, X²⁷ = Asn, X²⁸ = Ile, X²⁹ = Cys and X³⁰ = Trp, Y is a spacer and m is 0 or 1, and the monomeric building blocks are linked by -CONR¹- or -NR¹CO- bonds in which R¹ in each case independently denotes hydrogen, methyl or ethyl, and pharmaceutically acceptable salts and derivatives thereof,
with the proviso that at least one of the monomeric building blocks X²¹-X²⁹ of the lead structure is replaced by one of the amino acid residues listed below:
X²¹: Asp, Glu, 2,3-diaminopropionic acid (Dap), 2,4-diaminobutyric acid (Dab), D-penicillanine (D-Pen), allylglycine (Alg), ornithine (Orn), Lys;
X²²: Asp, Glu;
X²³: Dab, Dap, His, citrulline (Cit), homocitrulline (Hci), norleucine (Nle);
X²⁴: homophenylalanine (Hph), 1,2,3,4-tetrahydroisoquinoline-3-carboxylic acid (Tic), thienylalanine (Thi), Trp, phenylglycine (Phg), 1-napthylalanine (1-Nal), 2-naphthylalanine (2-Nal), cyclohexylalanine (Cha);
X²⁵: Trp, Tic, Thi, Hph, Phg;
X²⁶: Val;
X²⁷: Asp, Glu;
X²⁸: Cha, 2-aminobutyric acid (Abu), tert.-leucine (Tle), α-aminoisobutyric acid (Aib);
X²⁹: Asp, Glu, Dap, Dab, Alg, D-Pen, Om, Lys.

2. Compounds as claimed in claim 1,
**characterized in that**
at least one of the monomeric building blocks X²¹, X²³, X²⁴, X²⁵, X²⁷ and X²⁸ of the lead structure has one of the meanings listed below:
X²¹: D-Pen;
X²³: Dap, Dab, Cit, Hci, Nle, His;
X²⁴: Thi, Hph, Phg, 1-Nal, 2-Nal, Cha;
X²⁵: Thi;
X²⁷: Asp;
X²⁸: Val, Cha

3. Compounds as claimed in claim 1
**characterized in that**
at least one of the monomeric building blocks X²¹, X²³, X²⁴, X²⁵ and X²⁸ of the lead structure has one of the meanings listed below:
X²¹: D-Pen;
X²³: Dab, Nle, Cit, Hci;
X²⁴: 1-Nal, 2-Nal, Cha;
X²⁵: Thi;
X²⁸: Cha

4. Compounds of the general structural formula (I): 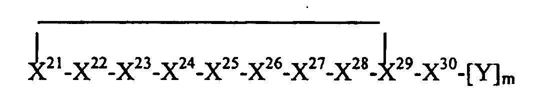 wherein X²¹-X³⁰ denote monomeric building blocks, preferably aminocarboxylic acid residues and are derived from a structure in which X²¹ = D-Cys, X²² = Asn, X²³ = Dap, Dab or Nle, X²⁴ = Tyr, X²⁵ = Phe, X²⁶ = Ser, X²⁷ = Asn, X²⁸ = Ile, X²⁹ = Cys and X³⁰ = Trp, Y is a spacer and m is 0 or 1, and the monomeric building blocks are linked by -CONR¹- or -NR¹CO- bonds in which R¹ in each case independently denotes hydrogen, methyl or ethyl, and pharmaceutically acceptable salts and derivatives thereof.

5. Compounds as claimed in one of the previous claims,
**characterized in that**
at least 2 of the amino acid residues X²², X²³, X²⁴, X²⁵, X²⁶, X²⁷, X²⁸ and X³⁰ have the same side chain as an amino acid at the same position in the native uPA sequence.

6. Compounds as claimed in claim 5,
**characterized in that**
at least 2 of the amino acid residues X²⁴, X²⁵, X²⁸ and X³⁰ have the same side chain as in the native uPA sequence.

7. Pharmaceutical composition which contains at least one compound as claimed in one of the claims 1 to 6 as the active substance optionally together with pharmaceutically common carrier substances, auxiliary substances or diluents.

8. Use of a compound as claimed in one of the claims 1 to 6 to produce a uPAR antagonist.

9. Use of a compound as claimed in claim 8 to produce an agent for treating diseases associated with the expression of uPAR and in particular for treating tumours.

10. Use of a compound as claimed in one of the claims 1 to 6 to produce a targeting vehicle for uPAR-expressing cells.

11. Use of a compound as claimed in one of the claims 1 to 6 to produce an angiogenesis inhibitor.

## Revendications

1. Composés de formule structurale générale (I) : 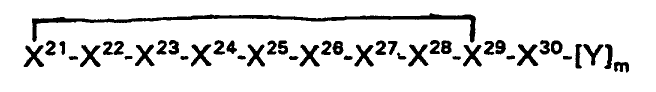 dans laquelle X²¹-X³⁰ représentent des éléments monomères, de préférence des résidus acides aminocarboxyliques, et sont dérivés d'une structure ayant la signification X²¹ = D-Cys, X²² = Asn, X²³ = Lys, X²⁴ = Tyr, X²⁵ = Phe, X²⁶ = Ser, X²⁷ = Asn, X²⁸ = Ile, X²⁹ = Cys et X³⁰ = Trp, Y est un espaceur et m est 0 ou 1, et les éléments monomères sont reliés par l'intermédiaire de liaisons -CONR¹- ou -NR¹CO-, où R¹ représente chaque fois indépendamment l'hydrogène, méthyle ou éthyle, et leurs sels et dérivés pharmaceutiquement acceptables,
à condition qu'au moins l'un des éléments monomères X²¹-X²⁹ de la structure de base soit remplacé par l'un des résidus d'aminoacides indiqués ci-dessous :
X²¹: Asp, Glu, acide 2,3-diaminopropionique (Dap), acide 2,4-diaminobutyrique (Dab), D-pénicillanine (D-Pen), allylglycine (Alg), omithine (Orn), Lys ;
X²² : Asp, Glu;
X²³ : Dab, Dap, His, citrulline (Cit), homocitrulline (Hci), norleucine (Nle) ;
X²⁴ : homophénylalanine (Hph), acide 1,2,3,4-tétrahydroisoquinoléine-3-carboxylique (Tic), thiénylalanine (Thi), Trp, phénylglycine (Phg), 1-naphtylalanine (1-Nal), 2-naphtylalanine (2-Nal), cyclohexylalanine (Cha) ;
X²⁵ : Trp, Tic, Thi, Hph, Phg ;
X²⁶ : Val ;
X²⁷ : Asp, Glu ;
X²⁸ : Cha, acide 2-aminobutyrique (Abu), tert-leucine (Tle), acide α-aminoisobutyrique (Aib) ;
X²⁹ : Asp, Glu, Dap, Dab, Alg, D-Pen, Orn, Lys.

2. Composés selon la revendication 1, **caractérisés en ce qu'**au moins l'un des éléments monomères X²¹, X²³, X²⁴, X²⁵, X²⁷ et X²⁸ de la structure de base possède l'une des significations indiquées ci-dessous :
X²¹ : D-Pen ;
X²³ : Dap, Dab, Cit, Hci, Nle, His ;
X²⁴ : Thi, Hph, Phg, 1-Nal, 2-Nal, Cha ;
X²⁵ : Thi ;
X²⁷ : Asp ;
X²⁸ : Val, Cha.

3. Composés selon la revendication 1, **caractérisés en ce qu'**au moins l'un des éléments monomères X²¹, X²³, X²⁴, X²⁵ et X²⁸ de la structure de base possède l'une des significations indiquées ci-dessous :
X²¹ : D-Pen ;
X²³ : Dab, Nle, Cit, Hci ;
X²⁴ : 1-Nal, 2-Nal, Cha ;
X²⁵ : Thi ;
X²⁸ : Cha.

4. Composés de formule structurale générale (I) : 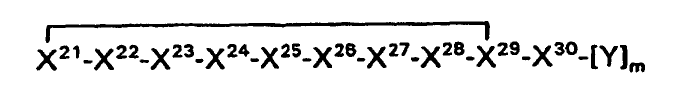 dans laquelle X²¹-X³⁰ représentent des éléments monomères, de préférence des résidus acides aminocarboxyliques, et sont dérivés d'une structure ayant la signification X²¹ = D-Cys, X²² = Asn, X²³ = Dap, Dab ou Nle, X²⁴ = Tyr, X²⁵ = Phe, X²⁶ = Ser, X²⁷ = Asn, X²⁸ = Ile, X²⁹ = Cys et X³⁰ = Trp, Y est un espaceur et m est 0 ou 1, et les éléments monomères sont reliés par l'intermédiaire de liaisons -CONR¹- ou -NR¹CO-, où R¹ représente chaque fois indépendamment l'hydrogène, méthyle ou éthyle, et leurs sels et dérivés pharmaceutiquement acceptables,

5. Composés selon l'une des revendications précédentes, **caractérisés en ce qu'**au moins 2 des résidus aminoacides X²², X²³, X²⁴, X²⁵, X²⁶, X²⁷, X²⁸ et X³⁰ présentent la même chaîne latérale qu'un aminoacide à la même position dans la séquence de l'uPA natif.

6. Composés selon la revendication 5, **caractérisés en ce qu'**au moins 2 des résidus aminoacides X²⁴, X²⁵, X²⁸ et X³⁰ présentent la même chaîne latérale que dans la séquence de l'uPA natif.

7. Composition pharmaceutique contenant comme substance active au moins un composé selon l'une des revendications 1 à 6, éventuellement avec des supports, substances auxiliaires ou diluants classiques en pharmacie.

8. Utilisation d'un composé selon l'une des revendications 1 à 6 pour la préparation d'un antagoniste de l'uPAR.

9. Utilisation d'un composé selon la revendication 8 pour la préparation d'un agent destiné à lutter contre des maladies associées à l'expression de l'uPAR, en particulier contre des tumeurs.

10. Utilisation d'un composé selon l'une des revendications 1 à 6 pour la préparation d'un véhicule de ciblage pour des cellules exprimant de l'uPAR.

11. Utilisation d'un composé selon l'une des revendications 1 à 6 pour la préparation d'un inhibiteur de l'angiogenèse.
